# EUROPEAN PATENT APPLICATION

(11) **EP 4 529 866 A2**
(43) Date of publication of application: **02.04.2025**
(21) Application number: 25158167.4
(22) Date of filing: 10.12.2021
(51) Int. Cl.: A61B 17/32

(54) **CUTTING ASSEMBLY WITH IRRIGATION AND ASPIRATION**

(30) Priority: 11.12.2020 US 202063124207 P
(62) Divisional of application: 21827659.0
(71) Applicant: Stryker European Operations Limited, Carrigtwohill, Co. Cork T45 HX08 (IE)
(72) Inventor: BROWNE, Jonathan, Dublin, 18 (IE); O'SHEA, Conor, Innishannon (IE); CURTIN, Damian Michael, Tralee Co. Kerry (IE); WALSH, Joshua, Cork Midleton, P25KD85 (IE)
(74) Representative: Röthinger, Rainer

(57) **Abstract**

A cutting assembly (10) configured to be coupled to an irrigation source and an aspiration source is presented. The cutting assembly (10) comprises a housing (12) and an outer tube (16) comprising a proximal end coupled to the housing (12) and comprising an outer tip portion (36) defining an outer window (42). The cutting assembly (10) further comprises an inner tube (18) rotatably and coaxially disposed within the outer tube (16) and defining an aspiration lumen (24) configured to be in arranged in fluid communication with the aspiration source. The inner tube (18) comprises an inner tip portion (38) defining an inner window (46). Further, a gap (58) is defined between the inner tip portion (38) and the outer tip portion (36). The outer tube (16) defines irrigation channels (60) extending longitudinally from near the proximal end and configured to be arranged in fluid communication with the irrigation source, and further defines irrigation apertures (62) configured to provide fluid communication between the irrigation channels (60) and the gap (58) at a position proximal to the outer window (42) and the inner window (46).

## Description

### PRIORITY CLAIM

This application claims priority to and all the benefits of United States Provisional Application No. 63/124,207, filed December 11, 2020, the entire contents of which are hereby incorporated by reference.

### BACKGROUND

Angled cutting instruments for surgery facilitate treatment of anatomy not otherwise accessible to straight instrumentation. The angled cutting instruments typically include a drive shaft or inner tube rotatably disposed within an outer tube, and require transmission of torque along or about a bend. Angled shavers may provide for aspiration, often through an aspiration lumen defined by the inner tube. Yet the geometries often necessary to facilitate the transmission of torque about the bend render the aspiration lumen suspectable to undesirable ingress or egress of fluid through the geometries. One such cutting instrument is an angled shaver described in United States Patent No. 8,623,266 to Adams, in which a heat shrunk sleeve is disposed over a continuous helical cut of an inner tube. The heat shrunk sleeve is susceptible to wear when rotated at high speed in a journal bearing arrangement with the outer tube. Another known angled shaver is described in United States Patent No. 5,922,003 to Anctil et al., in which a portion of the inner tube is replaced with a length of flexible coupling. The arrangement undesirably requires complex means of fabrication to effectively overmold the flexible coupling with lap joints of adjacent components. Further, the capacity for the flexible coupling to effectively transmit torques required of certain cutting operations is suspect. Still further, certain flexible couplings may tend to kink when bent, or alternatively those being sufficiently study are typically too large for most desired surgical applications. Therefore, there is a need in the art for an improved cutting assembly that provides for irrigation and aspiration and about a bend.

### SUMMARY

A cutting assembly may include an aspiration path for suctioning resected tissue and other surgical debris, and an irrigation path for irrigating the surgical site. A movable or rotatable inner tube may define at least a portion of the aspiration path, and the irrigation path may be external to the inner tube. The cutting assembly includes means for preventing ingress of fluid from within the irrigation path to within the aspiration path, thereby maximizing irrigation and suction capabilities. The cutting assembly may include a tube assembly that is straight or angled. The means for preventing ingress of the fluid may be particularly well suited for angled cutting assemblies. The cutting assembly may be a shaver, a bur, or other tissue manipulating device in which suction and irrigation is incorporated. Alternatively, the means for preventing fluid ingress may also be used on instruments with irrigation but no suction, or with suction but no irrigation.

The cutting assembly includes a housing hub, and the tube assembly is coupled to the housing hub. The tube assembly includes at least an outer tube, and the inner tube is movably or rotatably disposed within the outer tube. A drive hub is rigidly coupled to the inner tube and includes keys and other features configured to releasably engage complementary components of a motor and capital equipment. The drive hub and the inner tube may define an aspiration lumen in fluid communication with a cutting tip disposed at a distal end of the tube assembly. The aspiration lumen may define the aspiration path. The resected tissue and other debris and fluids are drawn through the cutting tip. In certain implementations, the materials being aspirated through the inner tube traverse a bend of the tube assembly to pass through the housing hub for collection through the capital equipment. The housing hub defines an irrigation cavity or lumen. A seal may be coupled to the housing hub and defines an opening in fluid communication with the irrigation cavity. The seal is configured to be positioned in a sealing relationship with a complementary feature of the capital equipment to prevent egress of fluid during irrigation being supplied through the surgical instrument. The tube assembly extends distally from the housing hub. The outer tube may be rigidly coupled to the housing hub. The inner tube is rotatable within the outer tube, and thus is rotatable relative to the housing hub. With the drive hub of the cutting assembly coupled to the capital equipment, relative axial movement between the inner and outer tubes is prevented.

In certain implementations, the cutting tip defines a cutting window such that the cutting assembly is a shaver. The cutting assembly may include an outer tip portion and an inner tip portion. The outer tip portion is rigidly coupled to the outer tube. The outer tip portion includes a collar configured to be welded to the outer tube. Distal and relative to an outer diameter of the collar, the outer tip portion may include a thinned region. The thinned region has an outer diameter less of that than the collar. The thinned region may be formed through a plunge grind or other suitable manufacturing technique. The thinned region may be tapered, for example, in a direction towards an outer window defined by the outer tip portion.

Cutting teeth may be disposed adjacent the outer window. The inner tip portion is rigidly coupled to the inner tube. The inner tip portion defines an inner window, and cutting teeth may be disposed adjacent the inner window. Disposed within the inner tip portion may be an element for minimizing clogging of the cutting assembly. A gap may be defined between the outer tip portion and the inner tip portion. The gap provides clearance not only for the inner tip portion to rotate within the outer tip portion, but also for the irrigation to discharge from the cutting assembly at the surgical site. The fluid may be directed through the gap, and discharged through a periphery of the outer window.

The bend of the tube assembly may include a bend of the outer tube, and a flexible region of the inner tube. The outer tube may be rigid or malleable. Another tube may be coaxially disposed over the outer tube with the resulting arrangement providing for rotation of the cutting window independently of the bend of the tube assembly. The flexible region may define slots. In one example, the flexible region includes segments interlocked with one another to define the slots. The segments may be castellated as shown, and other interlocking geometries are contemplated. The segments may be present for an entirety of the inner tube, or present for a portion of the inner tube at least including the flexible region. Alternatively, the inner tube may include helical, T-slots, windings, braids, or the like, to transmit torque about the bend.

In certain implementations, the outer tube defines irrigation channels extending longitudinally along a length of the outer tube. The irrigation channels are in fluid communication with the irrigation cavity of the housing hub. The irrigation channels may be defined by the outer tube itself. The outer tube may be monolithic in construction and encapsulate the irrigation channels. Alternatively, the outer tube may define longitudinal slots or recesses, and a hypotube or intermediate tube may be secured within the outer tube to define the irrigation channels. The arrangement provides of the irrigation channels defining an irrigation path that is fluidly separated from the aspiration lumen. The irrigation fluid traverses the bend towards the gap without risk of being undesirably drawn into the aspiration lumen through the slots between the segments. Any number of the irrigation channels are contemplated, and the irrigation channel(s) may be located in any suitable radial arrangement.

The outer tube further defines irrigation apertures providing fluid communication between the irrigation channels and the gap defined between the inner and outer tip portions. The irrigation apertures may be slots defined by an inner surface of the outer tube. The irrigation apertures may be formed by inner thickness(es) of the outer tube terminating such that the irrigation channels effectively open into the gap. Any number of the irrigation apertures are contemplated, and the irrigation aperture(s) may be located in any suitable radial arrangement. The irrigation apertures are positioned distal to the bend. The irrigation apertures may be positioned distal to a distalmost one of the segments. The irrigation channels may be positioned near or adjacent the inner and outer tip portions. The irrigation apertures redirect the fluid from the irrigation channels to the gap distal to the bend but proximate to the cutting tip. The irrigation fluid is discharged from the cutting assembly through the cutting tip.

Fluid communication is established between the irrigation channels and the irrigation cavity of the housing hub. The housing hub may define an irrigation aperture through which the inner tube extends. The housing hub may further define recesses extending from the irrigation aperture and in rotational alignment with the irrigation channels. The arrangement provides for the irrigation fluid entering the irrigation cavity of the housing hub being further directed through the and the irrigation channels. In an alternative implementation, the recesses and/or the irrigation channels may provide for fluid communication in any rotational alignment. There may be more than four recesses, and/or the recesses and the irrigation channels may subtend larger arcs such that, in any rotational orientation between the tube assembly and/or the housing hub, at least a portion of one of the irrigation channels is in fluid communication with at least a portion of one of the recesses.

In certain implementations, the tube assembly includes an inner jacket coaxially disposed within the inner tube. The inner jacket may have an outer diameter approximate to an inner diameter of the inner tube. The inner jacket may be considered a sleeve or liner. The inner jacket has mechanical properties configured to allow the inner tube to remain flexible along the bend, and further provide a seal within the aspiration lumen to prevent aspiration of the irrigation fluid through the slots of the segments. The inner jacket may or may not facilitate the transmission of torque. The inner jacket may be a multilayered and reinforced tube. For example, the inner jacket includes braided wire disposed or sandwiched between polymeric layers. An inner layer and/or the outer layer of the inner jacket may be formed from polyether block amide, and the braided wire may be stainless steel. Alternatively, the inner jacket may be formed from polytetrafluoroethylene (PTFE). The braids may be extremely thin and ribbon-like in construction. In innermost layer may optionally be chemically etched within with the inner layer and formed from PTFE. The inner layer or the innermost layer may define a liner lumen that itself defines at least a portion of the aspiration lumen. The innermost layer being PTFE is lubricious and therefore reduces potential clogging as debris is pulled through the liner lumen.

The inner jacket includes a distal end positioned adjacent to the inner tip portion, and a proximal end coupled to the inner tube or the drive hub. The arrangement results in the inner jacket lining nearly an entirety of the aspiration lumen between the cutting tip and a proximal end of the drive hub. The inner tip portion may include a counterbore approximately sized to the thickness of the inner jacket. The distal end of the inner jacket is positioned within the counterbore. The proximal end of the inner jacket may be positioned proximal to a proximal end of the inner tube. A portion of the inner jacket proximal to the inner tube may be coupled to the drive hub with an adhesive or other suitable joining means. The proximal end and the distal end of the inner jacket are positioned opposite the flexible region of the inner tube. The inner jacket provides the seal along the bend or curve so as to prevent aspiration of the irrigation fluid through the slots of the segments.

In certain implementations, the cutting assembly may be a bur. The cutting tip may be a bur head secured to the inner tube. The outer tube may terminate at a tubular distal end, and the inner tube may extend through the tubular distal end. A neck of the cutting tip may extend from the bur head and define an aperture positioned adjacent and proximal to the bur head with the aperture in fluid communication with the aspiration lumen. The inner tube is coupled to the cutting tip. A distal end of the inner tube may be secured to a proximal end of the neck of the cutting tip. The inner jacket is coaxially disposed within the inner tube. The proximal end and the distal end of the inner jacket are configured to at least be positioned opposite the flexible region of the inner tube in which there are slots, and consequently the inner jacket assumes a complementary bend or curve. The distal end of the inner jacket may be positioned adjacent to the proximal end of the neck. The proximal end of the inner jacket may be positioned proximal to the proximal end of the inner tube. A portion of the inner jacket proximal to the inner tube may be coupled to the drive hub. The distal end of the inner jacket may or may not be coupled to the cutting tip. Implementations including the inner jacket may be combined with implementations including the irrigation channels and apertures.

The cutting assembly may further include an irrigation spacer disposed within the housing hub and distal to the drive hub. The irrigation spacer may be disposed in a distal cavity extending distally from the irrigation cavity. In one implementation, the irrigation spacer may have a hub that defines a bore through which the inner tube and the inner jacket extend, and fins extending radially away from the hub. At least one washer may be positioned between the irrigation spacer and the drive hub. The irrigation spacer provides for axial spacing of the drive hub from the irrigation aperture while also providing irrigation passageways to permit robust fluid flow through the irrigation path.

Therefore, a first aspect of the present disclosure is directed to the cutting assembly configured to be coupled to the irrigation source and the aspiration source. The cutting assembly includes the housing, the outer tube, the inner tube, and the cutting tip. The proximal end of the outer tube is coupled to the housing. The inner tube coupled to the drive hub and rotatably and coaxially disposed within the outer tube. The irrigation path is defined between the inner tube and the outer tube. The cutting tip is secured to the inner tube. The inner jacket is coupled to the drive hub and coaxially disposed within the inner tube. The aspiration path is defined within the inner jacket. The inner jacket is configured to provide a fluid seal between the aspiration path and the irrigation path.

In certain implementations, the inner tube may define slots. The fluid seal provided by the inner jacket is configured to prevent ingress of irrigation fluid through the slots. The outer tube may include a bend, and the slots of the inner tube may be axially positioned along the bend. The inner jacket may be coupled to the drive hub at a position proximal to where a proximal end of the inner tube is coupled to the drive hub. The inner jacket may not be secured to the cutting tip. The inner jacket may a multilayer reinforced tube, such as a braid disposed between inner and outer layers of polymeric material. The braid may be stainless steel, and the polymeric material may be polyether block amide.

In certain implementations, the outer tube includes the outer tip portion defining the outer window, and the inner tip portion comprises the inner window such that the cutting assembly is a shaver. Alternatively, the cutting tip may be a bur head. The bur head may include a cutting element and define an aspiration port proximal to the cutting element. An irrigation spacer may be disposed within a cavity defined by the housing. The irrigation spacer may include a hub defining a bore through which the inner tube is rotatably disposed, and fins extending radially from the hub to be secured within the cavity.

According to a second aspect of the present disclosure, the cutting assembly includes the housing, the outer tube, and the inner tube. The proximal end of the outer tube is coupled to the housing and includes the outer tip portion defining an outer window. The inner tube is rotatably and coaxially disposed within the outer tube and defines the aspiration lumen configured to be in arranged in fluid communication with the aspiration source. The inner tube includes the inner tip portion defining the inner window, and a gap is defined between the inner tip portion and the outer tip portion. The outer tube defines irrigation channels extending longitudinally from near the proximal end and configured to be arranged in fluid communication with the irrigation source. The outer tube further defines irrigation apertures configured to provide fluid communication between the irrigation channels and the gap at a position proximal to the outer window and the inner window. The irrigation apertures may be positioned proximal to the outer window. The outer tube may include a bend and the inner tube may include a flexible region. The irrigation apertures may be positioned distal to the flexible region.

According to a third aspect of the present disclosure, the cutting assembly includes the housing, the outer tube, and the inner tube. The outer tube includes a distal end, and a bend to angle the distal end relative to a longitudinal axis. The inner tube is rotatably and coaxially disposed within the outer tube and includes the flexible region traversing the bend of the outer tube. The cutting tip is secured to the inner tube and angled relative to the longitudinal axis. The outer tube defines the irrigation channels configured to be in fluid communication with the irrigation source. The irrigation channels are fluidly separated from the flexible region of the inner tube. The outer tube further defines the irrigation apertures positioned distal to the flexible region and configured to provide fluid communication between the irrigation channels and the gap between the cutting tip and the outer tube.

In certain implementations, the outer tube may include the outer tip portion defining the outer window, and the cutting tip may define the inner window such that the cutting assembly is a shaver. Alternatively, the cutting tip may be a bur head. The outer tube may include the bend, and the irrigation channels may traverse the bend. The inner tube may include the segments defining the flexible region. The irrigation channels are fluidly separated from the segments. The outer tube may be monolithic, and the irrigation channels are encapsulated within the outer tube. Alternatively, a hypotube may be disposed within and coupled to the outer tube, wherein and the irrigation channels are defined between the outer tube and the hypotube. The irrigation apertures may be recesses defined at a distal end of the irrigation channels. The irrigation channels may be four irrigation channels radially spaced equally about the longitudinal axis, and the irrigation apertures may be four irrigation apertures radially spaced equally about the longitudinal axis. The housing may define an irrigation cavity, an aperture through which the inner tube extends, and recesses in fluid communication with the aperture and the irrigation channels. The c recesses of the housing may be in a cruciform or other suitable arrangement.

### BRIEF DESCRIPTION OF THE DRAWINGS

Advantages of the present disclosure will be readily appreciated as the same becomes better understood by reference to the following detailed description when considered in connection with the accompanying drawings.
FIG. 1 is a perspective view of a cutting assembly of a surgical instrument.
FIG. 2 is a sectional elevation view of the cutting assembly of FIG. 1 taken along section lines 2-2.
FIG. 2A is a detailed view of a portion of cutting assembly within circle 2A.
FIG. 3 is a sectional axial view of the cutting assembly of FIG. 1 taken along section lines 3-3.
FIG. 4 is a sectional axial view of the cutting assembly of FIG. 1 taken along section lines 4-4.
FIG. 5 is an exploded view of a distal portion of the cutting assembly. Inner and outer tip portions are shown decoupled from an inner tube and an outer tube, respectively, for visualization of irrigation apertures.
FIG. 6 is a sectional axial view of a housing of the cutting assembly of FIG. 1 taken along section lines 6-6.
FIG. 7 is a rear perspective view of a hub of the cutting assembly.
FIG. 8 is an exploded view of a cutting assembly according to another implementation in which an inner jacket is configured to be coaxially disposed within the inner tube.
FIG. 9 is a sectional elevation view of the inner and outer tip portions of FIG. 8 with the inner jacket coupled to the inner tip portion.
FIG. 10 is a sectional elevation view of the housing with the inner jacket coupled to a drive hub of the cutting assembly.
FIG. 11 is a perspective view of a cutting assembly of a surgical instrument.
FIG. 12 is a sectional elevation view of the cutting assembly of FIG. 11 taken along section lines 12-12.
FIG. 12A is a detailed sectional view of a portion of cutting assembly within circle 12A.
FIG. 13 is a detailed sectional view of another portion of cutting assembly within rectangle 13.
FIG. 14 is a detailed sectional view of another portion of cutting assembly within rectangle 13.

### DETAILED DESCRIPTION

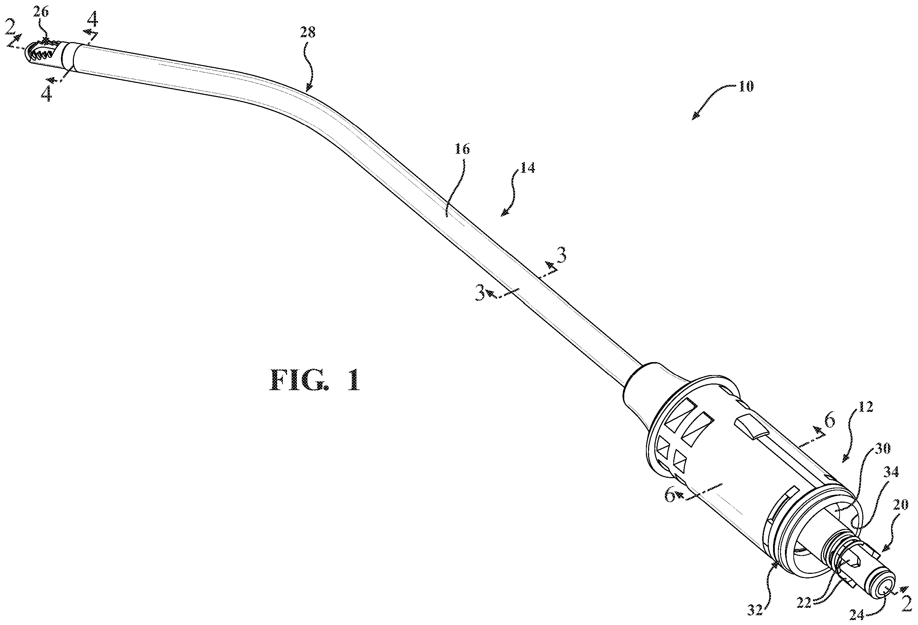

FIG. 1 shows a cutting assembly 10 configured to resect tissue. The cutting assembly 10 of FIG. 1 may be considered an angled shaver. The cutting assembly 10 is configured to be removably coupled to capital equipment (not shown) that may include one or more of a motor, an irrigation source, and an aspiration source. Whereas the capital equipment may be reused in multiple procedures, the cutting assembly 10 may be a disposable component. One exemplary capital equipment suitable for the present application is sold under the tradename ESSx Microdebrider manufactured by the Stryker Corporation (Kalamazoo, Mich.), and/or disclosed in commonly-owned United States Patent No. 6,152,941, issued November 28, 2000, and International Publication No. WO 2021/224862 published November 11, 2021, the entire contents of which are hereby incorporated by reference.

The cutting assembly 10 includes a housing hub 12, and a tube assembly 14 coupled to the housing hub 12. The tube assembly 14 includes at least an outer tube 16, and an inner tube 18 rotatably disposed within the outer tube 16. The inner tube 18 is configured to be coupled to the motor of the capital equipment, and further configured to be rotated within the outer tube 16 by the motor. A drive hub 20 is rigidly coupled to the inner tube 18 and includes keys 22 and other features configured to releasably engage complementary components of the capital equipment.

The drive hub 20 and the inner tube 18 may define an aspiration lumen 24 in fluid communication with a cutting tip 26 disposed at a distal end of the tube assembly 14. The aspiration lumen 24 may define an aspiration path. With the cutting assembly 10 removably coupled with the capital equipment, the aspiration path is configured to be arranged in fluid communication with the aspiration source. The tissue resected by relative rotation of the inner and outer tubes 16, 18 is drawn through the cutting tip 26, and other debris and fluids may similarly be aspirated from the surgical site. The materials being aspirated through the inner tube 18 traverse a bend 28 of the tube assembly 14 to pass through the housing hub 12 for collection through the capital equipment. As to be further described, aspirating fluids through a bend of a rotatable tube - and further providing irrigation through the tube assembly - is associated with technical challenges overcome with the cutting assembly 10 of the present disclosure.

The housing hub 12 defines an irrigation cavity 30 or lumen. A seal 32 may be coupled to the housing hub 12 and defines an opening 34 in fluid communication with the irrigation cavity 30. One seal suitable for the present application is disclosed in the aforementioned International Publication No. WO 2021/224862. The seal 32 is configured to be positioned in a sealing relationship with a complementary feature of the capital equipment to prevent egress of fluid during irrigation being supplied through the surgical instrument.

The tube assembly 14 extends distally from the housing hub 12. The outer tube 16 may be rigidly coupled to the housing hub 12. The inner tube 18 is rotatable within the outer tube 16, and thus is rotatable relative to the housing hub 12. With the drive hub 20 of the cutting assembly 10 coupled to the capital equipment, relative axial movement between the inner and outer tubes 16, 18 is prevented. With the cutting assembly 10 decoupled from the capital equipment, slight relative axial movement between the inner and outer tubes 16, 18 may be permitted.

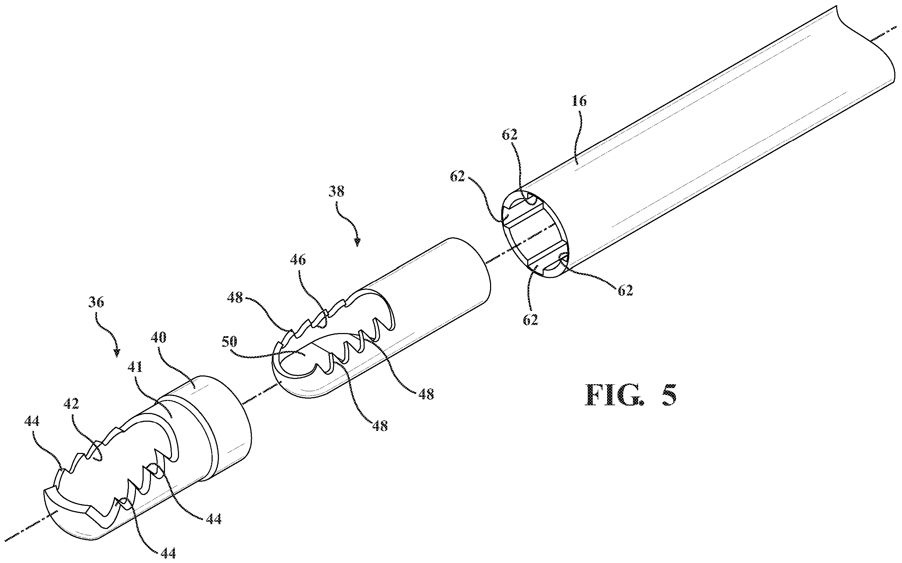

The cutting tip 26 may define a cutting window such that the cutting assembly 10 is a shaver. With further reference to FIG. 5, the cutting assembly 10 includes an outer tip portion 36 and an inner tip portion 38. The outer tip portion 36 is rigidly coupled to the outer tube 16. In one implementation, the outer tip portion 36 includes a collar 40 configured to be welded to the outer tube 16 (at interface shown in FIG. 5). Distal and relative to the collar 40, the outer tip portion 36 may include a thinned region 41. The thinned region 41 has an outer diameter less than the outer diameter of the collar 40. The thinned region 41 of the outer tip portion 36 provides for the cutting teeth 44 disposed adjacent the outer window 42 to be made sharper, and improve visibility of the surgeon of the cutting tip 26. The thinned region 41 may be formed through a plunge grind or other suitable manufacturing technique. The thinned region 41 may be tapered, for example, in a direction towards an outer window 42 defined by the outer tip portion 36.

The inner tip portion 38 is rigidly coupled to the inner tube 18. The inner tip portion 38 defines an inner window 46, and cutting teeth 48 may be disposed adjacent the inner window 46. The inner and outer windows 42, 46 may be considered to collectively define the cutting window of the cutting assembly 10. With the inner tip portion 38 being rotatably disposed within the outer tip portion 36, the cutting teeth 44, 48 cross one another in a shearing action to resect the tissue. Disposed within the inner tip portion 38 may be an element 50 for minimizing clogging of the cutting assembly 10 as disclosed in the aformentioned International Publication No. WO 2018/013906, published January 18, 2018, the entire contents of which is hereby incorporated by reference.

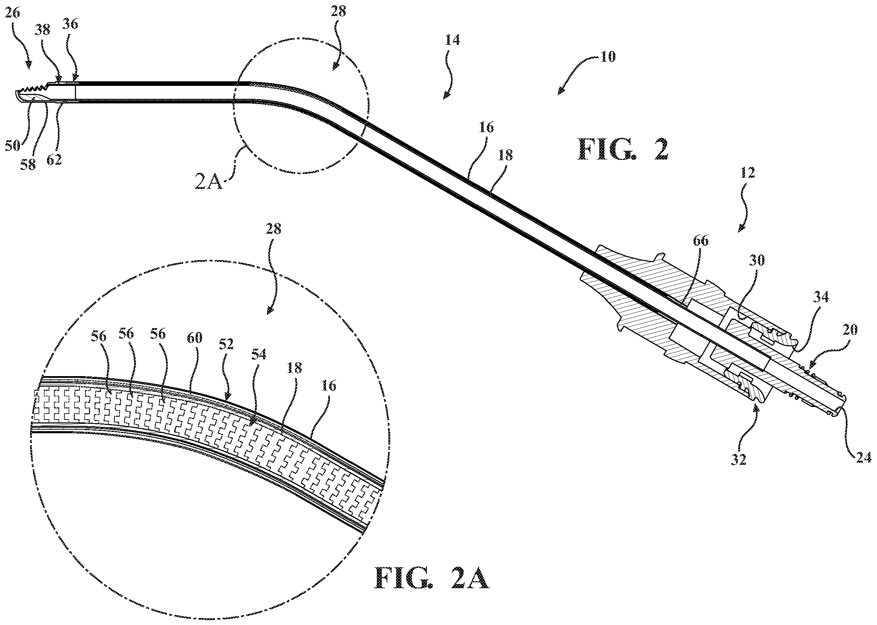

Referring now to FIG. 2 and 2A, the bend 28 of the tube assembly 14 may include a bend 52 of the outer tube 16, and a flexible region 54 of the inner tube 18. The outer tube 16 may be rigid or malleable, and thus the bend 52 of the outer tube 16 generally defines the angled shape of the tube assembly 14. The bend 28 may be formed at any suitable angle, which is often indicated by the type of procedure. For example, the angle may be 30°, 60°, or 90° or more limited only by the torque transmitting capabilities of the flexible region 54 of the inner tube 18. It is further understood that the bend 28 of the tube assembly 14 may be disposed at any axial location between the housing hub 12 and the cutting tip 26, and the bend 28 may be oriented in any radial direction relative to the housing hub 12 (*e.g.,* upwards, downwards, or laterally). These parameters may also be influenced by the type of procedure. For example, a proximal bend may be indicated for a spine surgery, a medial bend may be indicated for general thoracic surgery, and a distal bend may be indicated for otolaryngological surgery. FIG. 2 illustrates a distal bend at an angle of approximately 30° between opposing portions of the tube assembly 14 opposite the bend 28. Still further, it is contemplated that another tube may be coaxially disposed over the outer tube 16 with the resulting arrangement providing for rotation of the cutting window independently of the bend 28 of the tube assembly 14.

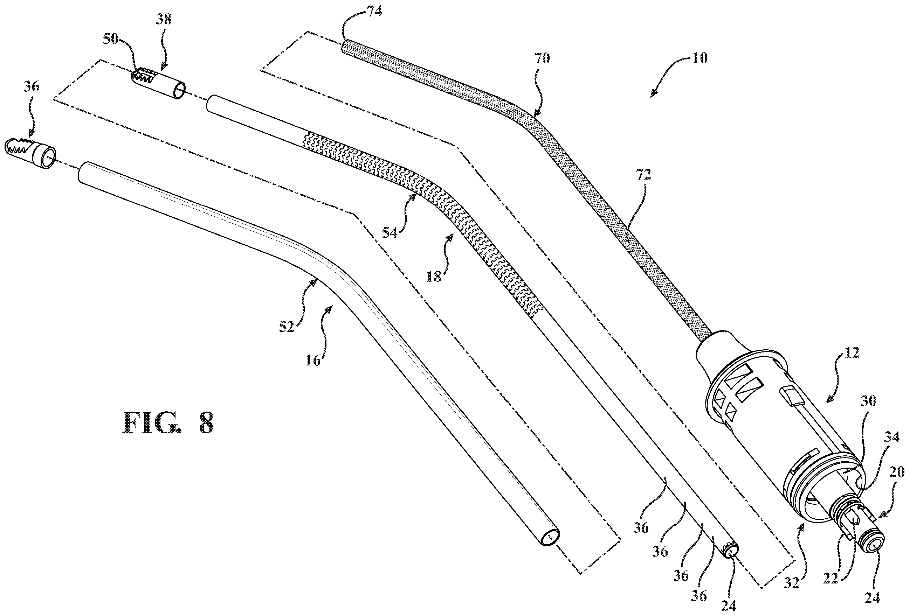

FIG. 2A best shows an exemplary implementation of the flexible region 54 of the inner tube 18. The flexible region 54 may define slots. In one example, the flexible region includes segments 56 interlocked with one another to define the slots. The segments 56 may be castellated as shown, however, other interlocking geometries are contemplated. The interlocking of the segments 56 provide for the transmission of torque with rotation of the inner tube 18 by the motor of the capital equipment. Additionally or alternatively, the inner tube 18 may include helical, wound, or braided characteristics configured to transmit torque about the bend 28. Relative to less intricate geometries, the segments 56 define smaller slots in which less loss of suction and/or less ingress of the irrigation fluid may occur. Despite the relatively smaller slots, improving suction performance of the cutting assembly 10 is of continued importance. This interest is particularly pronounced in implementations where the cutting assembly 10 also provide for irrigation through the tube assembly 14, often simultaneously with aspiration. It is contemplated that the segments 56 may be present for an entirety of the inner tube 18, or present for a portion of the inner tube 18 including the flexible region 54, as shown in FIG. 8. The extent to which the flexible region 54 forms the length of the inner tube 18 may be dependent on the flexural requirements of the tube assembly 14. For example, greater angles of bend and/or smaller radii of curvature may require a relatively greater length of the inner tube 18 being formed from the flexible region 54. In an exemplary implementation, the flexible region 54 forms less than 20% of the length of the tube assembly 14, and more particularly forms less than 20% of the length of the inner tube 18. Minimizing the distance of which the flexible region 54 forms of the length of the inner tube 18 may reduce whipping or chatter of the inner tube 18 when rotated at relatively high speeds. Further, a relatively smaller length of the flexible region 54 requires less segments 56, which better preserves transmission of torque from the drive hub 20 to the inner window 46. Despite the tolerances between the segments 56 being very small, cumulatively the stack up may result in some lag between the drive hub 20 and the inner window 46. Reducing the number of segments 56 may reduce any lag.

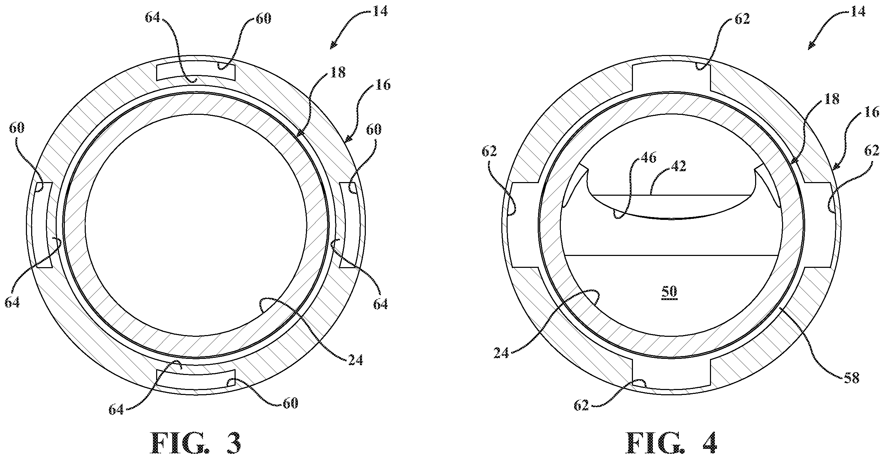

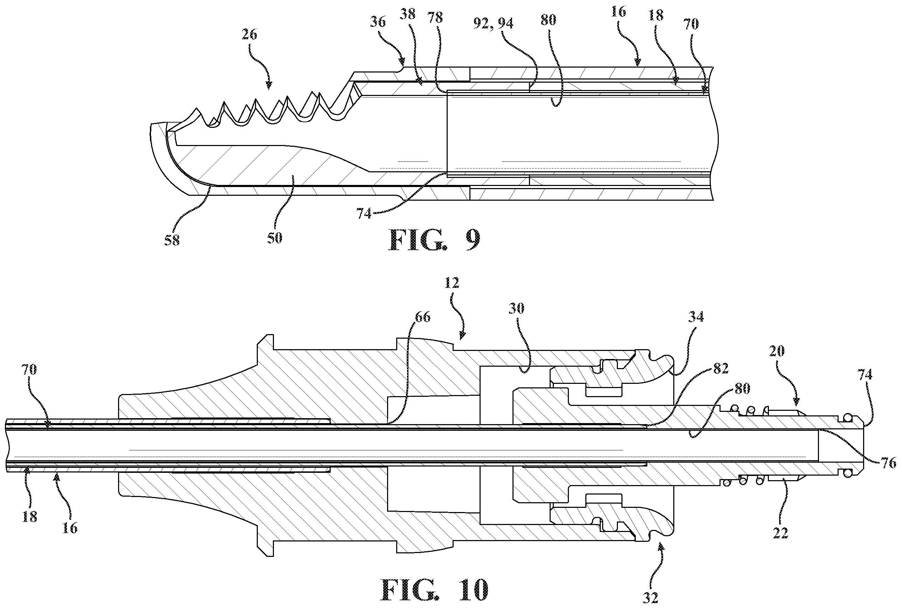

A gap 58 may be defined between the outer tip portion 36 and the inner tip portion 38 (best shown in FIG. 9B). The gap 58 provides clearance not only for the inner tip portion 38 to rotate within the outer tip portion 36, but also for the irrigation to discharge from the cutting assembly 10 at the surgical site. In particular, the fluid may be directed through the gap 58, and discharged through a periphery of the outer window 42. The gap 58 is configured to be placed in fluid communication with the irrigation source. With further reference to FIG. 3, the outer tube 16 defines irrigation channels 60 extending longitudinally along a length of the outer tube 16. The irrigation channels 60 are in fluid communication with the irrigation cavity 30 of the housing hub 12, and thus in fluid communication with the irrigation source when the cutting assembly 10 is coupled to the capital equipment. In an exemplary implementation shown in FIG. 3, the irrigation channels 60 are defined by the structure of the outer tube 16 itself. In other words, the outer tube 16 may be monolithic in construction and encapsulate the irrigation channels 60. The outer tube 16 may be formed with the irrigation channels 60 through an extrusion process. For another example, the outer tube 16 may define longitudinal slots or recesses, and a hypotube (not shown) or intermediate tube may be secured within the outer tube 16 to define the irrigation channels 60 with the slots. As best shown in FIG. 3, the arrangement provides of the irrigation channels 60 defining an irrigation path that is fluidly separated from the aspiration lumen 24, particularly along the bend 28. As a result, the irrigation fluid traverses the bend 28 towards the gap 58 without risk of being undesirably drawn into the aspiration lumen 24 through the slots between the segments 56. FIG. 3 shows four of the irrigation channels 60 radially spaced equiangularly about an axis of the tube assembly 14. More or less of the irrigation channels 60 are contemplated, and the irrigation channels 60 may be located in any suitable arrangement.

The outer tube 16 further defines irrigation apertures 62 providing fluid communication between the irrigation channels 60 and the gap 58 defined between the inner and outer tip portions 36, 38. Referring to FIGS. 4 and 5, the irrigation apertures 62 may be slots defined by an inner surface of the outer tube 16. In the illustrated implementation, the irrigation apertures 62 may be effectively formed by inner thickness(es) 64 of the outer tube 18 terminating (see FIG. 3) such that the irrigation channels 60 effectively open into the gap 58 as shown in FIG. 5. However, it is contemplated that the irrigation apertures 62 may take on any suitable geometry or form so as to provide fluid communication between the irrigation channels 60 and the gap 58. Further, the irrigation apertures 62 may be radially spaced equiangularly or in any suitable arrangement about an axis of the tube assembly 14. Further, more or less of the irrigation channels 60 are contemplated.

Because the irrigation channels 60 traverse the bend 28 with the irrigation channels 60 being fluidly separated from the inner tube 18 about the bend 28, the irrigation apertures 62 are positioned distal to the bend 28. The irrigation apertures 62 may be positioned distal to a distalmost one of the segments 56 such that the likelihood of irrigation fluid being aspirated through the slots is eliminated. In certain implementations, the irrigation channels 60 are positioned near or adjacent the inner and outer tip portions 36, 38. As such, the irrigation apertures 62 redirects the fluid from the irrigation channels 60 to the gap 58 distal to the bend 28 but proximate to the cutting tip 26. The irrigation fluid is discharged from the cutting assembly 10 through the cutting tip 26.

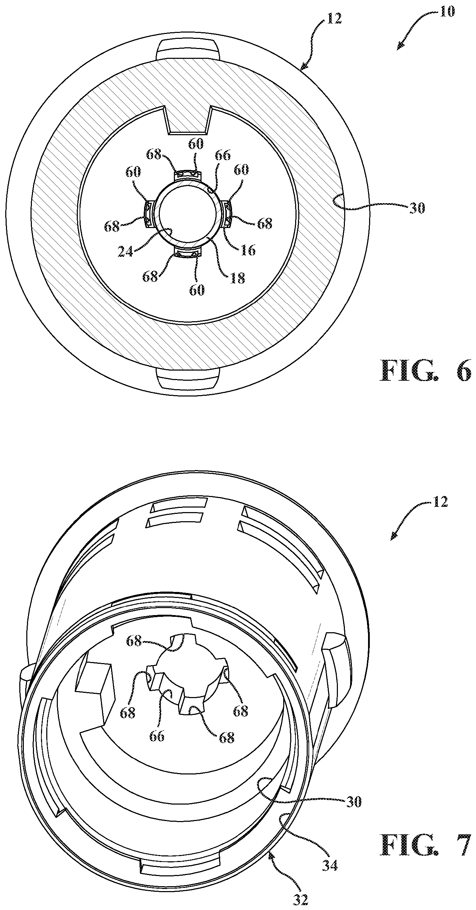

FIGS. 6 and 7 illustrate the fluid communication being established between the irrigation channels 60 and the irrigation cavity 30 of the housing hub 12. As previously explained, the inner tube 18 is rotatable relative to the housing hub 12, and therefore the housing hub 12 defines an irrigation aperture 66 through which the inner tube 18 extends (see also FIG. 2). The housing hub 12 may further define recesses 68 extending from the irrigation aperture 66 and in alignment with the irrigation channels 60, as best shown in FIG. 6. More particularly, assembly of the cutting assembly 10 may require the outer tube 16 be rigidly coupled to the housing hub 12 in a single rotational orientation so as to rotationally align the recesses 68 and the irrigation channels 60. The cruciform arrangement provides for the irrigation fluid entering the irrigation cavity 30 of the housing hub 12 being further directed through the recesses 68 and the irrigation channels 60. The irrigation path being entirely fluidly separate from the aspiration path optimizes irrigation and aspiration performance, particularly in instrumentation that includes a bend. In alternative implementations, it is contemplated that the tube assembly 14 may be straight with remaining aspects of the cutting assembly 10 being as presently described herein. Such a straight implementation may not include the segments 56, and the inner tube 18 may be a rigid in construction.

In alternative implementations, certain modifications may be provided on either the tube assembly 14 and/or the housing hub 12 to improve fluid flow between the irrigation cavity 30 and the irrigation channels 60. For example, as opposed to the recesses 68 and the irrigation channels being rotationally aligned in a single rotational orientation, the recesses 68 and/or the irrigation channels 60 may provide for fluid communication in any rotational orientation. There may be more than four recesses 68, and/or the recesses 68 may subtend larger arcs than the implementation shown in FIG. 6. Additionally or alternatively, the irrigation channels 60, or at least a proximal opening into the irrigation channels 60 may also subtend relatively larger arcs such that, in any rotational orientation between the tube assembly 14 and/or the housing hub 12, at least a portion of one of the irrigation channels 60 is in fluid communication with at least a portion of one of the recesses 68. For another example, a proximal end of the outer tube 16 may include inlet apertures (not shown) in communication with the irrigation channels 60. The inlet apertures may be similar to the irrigation apertures 62 previously described with the inlet apertures extending radially inwardly from an outer diameter of the outer tube 16. Whereas the irrigation channels 60 shown in FIG. 6 are defined within the proximal edge or end of the outer tube 16, and perhaps of limited size, irrigation inlets are of greater size to permit greater flow rates of fluid from the irrigation cavity 30 to the irrigation channels 60. For still another example, a seal (not shown) may be disposed between the inner tube 18 and the inner tube 16 near or adjacent the proximal end of the tube assembly 14. The seal may be a dynamic seal configured to engage the inner tube 18 that is rotating. The seal prevents fluid from entering the annular space between the inner and outer tubes 16, 18, which may reduce the effectiveness of the irrigation fluid entering the irrigation channels 60. In other words, should a fraction of the irrigating fluid otherwise enter the annular space, less fluid is entering the irrigation channels 60. The seal positioned near the openings (or inlet apertures) prevents ingress of the irrigation fluid into the annular space, and therefore an entirety of the irrigation fluid is directed into the irrigation channels 60 for improved performance of the cutting assembly 10.

FIGS. 8-10 are directed to an alternative embodiment of the cutting assembly 10 in which the irrigation fluid is separated from potential fluid communication with the aspiration. With like numerals indicating like components, the tube assembly 14 includes the outer tube 16 having the bend 52, the inner tube 18 having the flexible region 54. The tube assembly 14 includes an inner jacket 70 coaxially disposed within the inner tube 18. More particularly, the inner jacket 70 may have an outer diameter approximate to an inner diameter of the inner tube 18. The inner jacket 70 may be considered a sleeve or liner.

The inner jacket 70 has mechanical properties configured to allow the inner tube 16 to remain flexible along the bend 28, and further provide a seal within the aspiration lumen 24 to prevent aspiration of the irrigation fluid through the slots of the segments 56. The inner jacket 70 may or may not facilitate the transmission of torque. Known thin-walled, mono-polymer may tend to kink when deployed in a bent configuration, or alternatively is too large for typical surgical instrumentation. The inner jacket 70 overcomes such shortcomings by being a multilayered and reinforced tube in which advantageously achieves smaller wall thickness without the aforementioned kinking. In one implementation, the inner jacket 70 includes braided wire disposed or sandwiched between polymeric layers. The braided wire 72 is shown in FIG. 8. More particularly, an inner layer and/or the outer layer of the inner jacket 70 may be formed from polyether block amide sold under the trademark PEBAX sold by Arkema S.A. (Colomes, France), and the braided wire 72 may be stainless steel. Alternatively, the inner jacket 70 may be formed from polytetrafluoroethylene (PTFE). The braids may be extremely thin and ribbon-like in construction. In innermost layer may optionally be chemically etched within with the inner layer and formed from PTFE. The inner layer or the innermost layer may define a liner lumen 80 that itself defines at least a portion of the aspiration lumen 24. The innermost layer being PTFE is lubricious and therefore reduces potential clogging as debris is pulled through the liner lumen 80.

Referring now to FIGS. 9 and 10, the inner jacket 70 includes a distal end 74 positioned adjacent to the inner tip portion 38, and a proximal end 76 coupled to the inner tube 18 or the drive hub 20. The arrangement results in the inner jacket 70 lining nearly an entirety of the aspiration lumen 24 between the cutting tip 26 and a proximal end of the drive hub 20. Owing to a thickness of the inner jacket 70, the inner tip portion 38 may include a counterbore 78 approximately sized to the thickness of the inner jacket 70. As shown in FIG. 9, the distal end 74 of the inner jacket 70 is positioned within the counterbore 78. With the distal end 74 disposed within the counterbore 78, a smooth transition is present from an inner surface of the inner tip portion 38 to the inner jacket 70. In other words, the arrangement prevents exposed lips or edges from the distal end 74 of the inner jacket 70 with which the aspirated material may otherwise be snagged. The proximal end 76 of the inner jacket 70 may be positioned proximal to a proximal end 82 of the inner tube 18, as shown in FIG. 10. A portion of the inner jacket 70 proximal to the inner tube 18 may be coupled to the drive hub 20 with an adhesive or other suitable joining means. In certain implementations, the inner jacket 70 may not be coupled to the inner tip portion 38 and/or the drive hub 20, but rather free-floating in the appropriate axial position. The axial position may be maintained by the engagement between the bend of inner jacket 70 complementary to each of the flexible region 54 of the inner tube 18 and the bend 52 of the outer tube 16. The proximal end 76 and the distal end 74 of the inner jacket 70 are positioned opposite the flexible region 54 of the inner tube 18, and consequently the inner jacket 70 assumes a complementary bend or curve. The inner jacket 70 provides the seal along the bend or curve so as to prevent aspiration of the irrigation fluid through the slots of the segments 56.

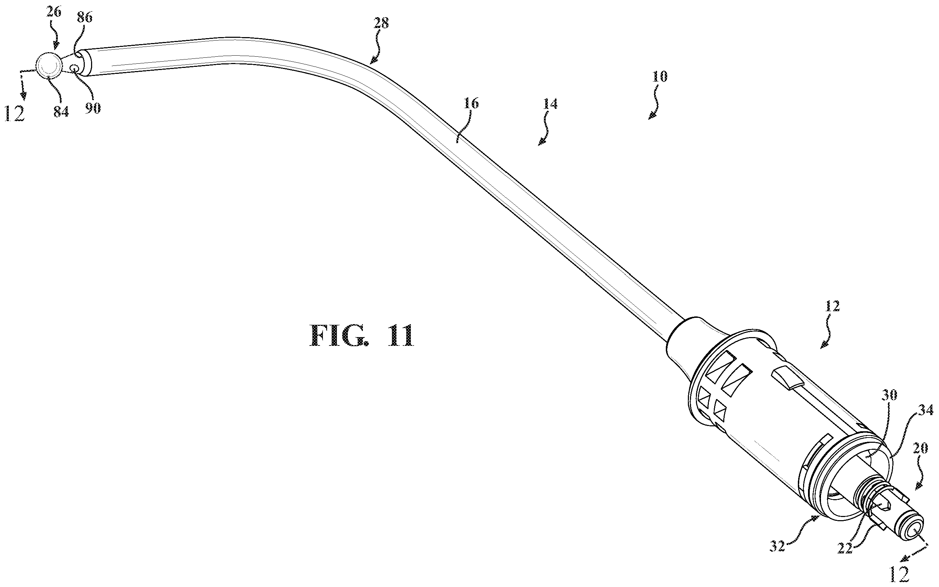

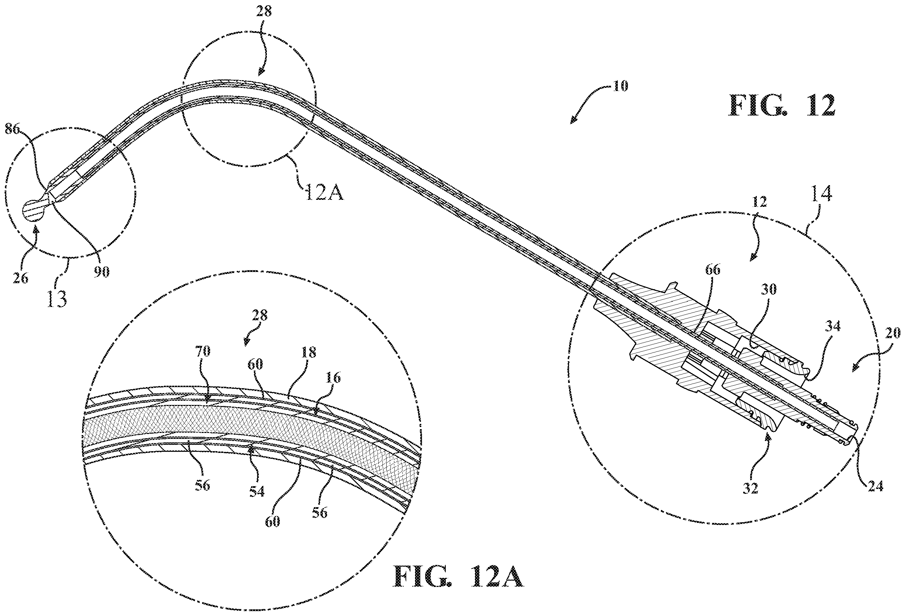

Referring now to FIGS. 11-14, the cutting assembly 10 may be a bur with like numerals indicating like components relative to the shaver of FIGS. 1-10. The cutting tip 26 may be a bur head 84 coupled to the inner tube 18. The outer tube 16 may not include the outer window 42, but rather terminate at a tubular distal end 86. The inner tube 18 may extend through the tubular distal end 86 for the bur head 84 to be positioned distal to the tubular distal end 86. A neck 88 of the cutting tip 26 may extend from the bur head 84 and define an aperture 90 positioned adjacent and proximal to the bur head 84 with the aperture 90 in fluid communication with the aspiration lumen 24. In alternative implementations, for example endoscopic applications, a partially shielded bur head may be rotatable within a window.

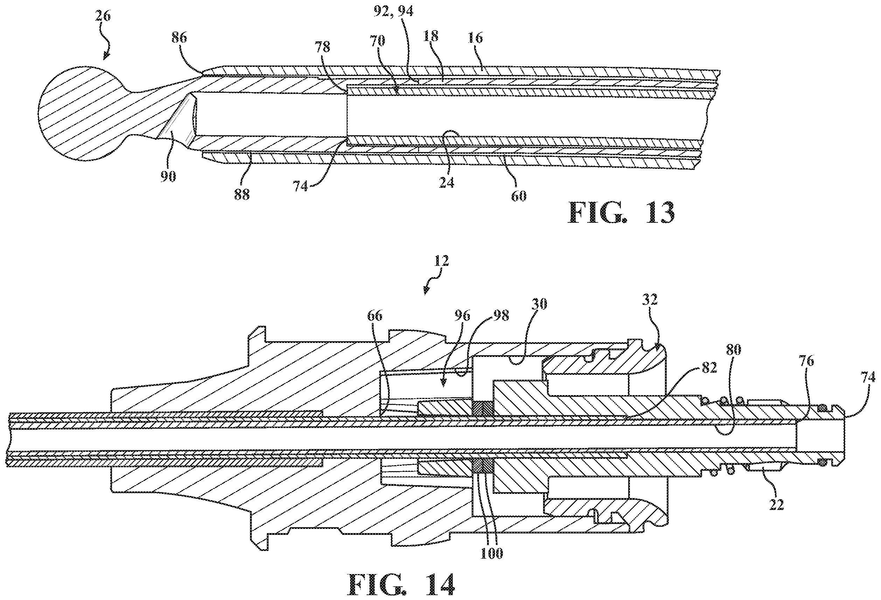

Like the implementation of the shaver described with reference to FIGS. 8-10, the cutting assembly 10 may include the outer tube 16, the inner tube 18, and the inner jacket 70. As best shown in FIG. 13, the inner tube 16 is coupled to the cutting tip 26, and more particularly a distal end 92 of the inner tube 16 is secured to a proximal end 94 of the neck 88 of the cutting tip 26. The distal end 92 of the inner tube 16 and the proximal end 94 of the neck 88 may be secured together by welding, brazing, or any other suitable joining means. The inner jacket 70 is coaxially disposed within the inner tube 16. The proximal end 76 and the distal end 74 of the inner jacket 70 are configured to at least be positioned opposite the flexible region 54 of the inner tube 18 in which there are slots defined by the segments 56, and consequently the inner jacket 70 assumes a complementary bend or curve. In certain implementations, the distal end 74 of the inner jacket 70 may be positioned adjacent to the proximal end 94 of the neck 88. The proximal end 76 of the inner jacket 70 may be positioned proximal to the proximal end 82 of the inner tube 18, as shown in FIG. 14. The arrangement results in the inner jacket 70 effectively lining nearly an entirety of the aspiration lumen 24 between the cutting tip 26 and the proximal end of the drive hub 20. A portion of the inner jacket 70 proximal to the inner tube 18 may be coupled to the drive hub 20 with an adhesive or other suitable joining means. The inner jacket 70 provides the seal along the bend or curve so as to prevent aspiration of the irrigation fluid through the slots of the segments 56. The distal end 74 of the inner jacket 70 may or may not be coupled to the cutting tip 26.

The cutting assembly 10 may further include an irrigation spacer 96 disposed within the housing hub 12 and distal to the drive hub 20. In implementations where the cutting assembly 10 is a shaver, interference between closed distal ends the outer tip portion 36 and the inner tip portion 38 prevents relative movement between the outer tube 16 and the inner tube 18. Such a constraint may not be present on a bur, and distal movement of the drive hub 20 within the irrigation cavity 30 may limit or occlude the flow of fluid through the irrigation aperture 66. The irrigation spacer 96 of the present implementation advantageously provides for axial spacing of the drive hub 20 from the irrigation aperture 66 while also providing irrigation passageways to permit robust fluid flow through the irrigation path. The irrigation spacer 96 may be disposed in a distal cavity 98 extending distally from the irrigation cavity 30, as shown in FIG. 14. In one implementation, the irrigation spacer 96 may have a hub that defines a bore through which the inner tube 18 and the inner jacket 70 extend, and fins extending radially away from the hub. At least one washer 100 may be positioned between the irrigation spacer 96 and the drive hub 20. Further disclosure of the irrigation spacer 96 and related components are disclosed in the aforementioned International Publication No. WO 2021/224862.

The foregoing description is not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

**The following examples are also encompassed by the present disclosure and may fully or partly be incorporated into embodiments. In the following, claims means embodiments:**
1. A cutting assembly configured to be coupled to an irrigation source and an aspiration source, the cutting assembly comprising:
   a housing;
   an outer tube comprising a proximal end coupled to the housing;
   a drive hub;
   an inner tube coupled to the drive hub and rotatably and coaxially disposed within the outer tube, wherein an irrigation path is defined between the inner tube and the outer tube;
   a cutting tip secured to the inner tube; and
   an inner jacket coupled to the drive hub and coaxially disposed within the inner tube, wherein an aspiration path is defined within the inner jacket, and wherein the inner jacket is configured to provide a fluid seal between the aspiration path and the irrigation path.
2. The cutting assembly of claim 1, wherein the inner tube defines slots, wherein the fluid seal provided by the inner jacket is configured to prevent ingress of irrigation fluid through the slots.
3. The cutting assembly of claim 1 or 2, wherein the outer tube comprises a bend, wherein the slots of the inner tube are axially positioned along the bend.
4. The cutting assembly of any one of claims 1-3, wherein the inner jacket is coupled to the drive hub at a position proximal to where a proximal end of the inner tube is coupled to the drive hub.
5. The cutting assembly of any one of claims 1-4, wherein the inner jacket is not secured to the cutting tip.
6. The cutting assembly of any one of claims 1-5, wherein the inner jacket is a multilayer reinforced tube.
7. The cutting assembly of claim 6, wherein the multilayer reinforced tube comprises a braid disposed between inner and outer layers of polymeric material.
8. The cutting assembly of claim 7, wherein the braid is stainless steel, and the polymeric material is polyether block amide.
9. The cutting assembly of any one of claims 1-8, wherein the outer tube comprises an outer tip portion defining an outer window, and an inner tip portion comprises an inner window such that the cutting assembly is a shaver.
10. The cutting assembly of any one of claims 1-9, wherein the cutting tip is a bur head.
11. The cutting assembly of claim 10, wherein the bur head comprises a cutting element and defines an aspiration port proximal to the cutting element.
12. The cutting assembly of claim 10 or 11, further comprising an irrigation spacer disposed within a cavity defined by the housing, the irrigation spacer comprising a hub defining a bore through which the inner tube is rotatably disposed, and fins extending radially from the hub to be secured within the cavity.
13. A cutting assembly configured to be coupled to an irrigation source and an aspiration source, the cutting assembly comprising:
   a housing;
   an outer tube comprising a proximal end coupled to the housing and comprising an outer tip portion defining an outer window; and
   an inner tube rotatably and coaxially disposed within the outer tube and defining an aspiration lumen configured to be in arranged in fluid communication with the aspiration source, the inner tube comprising an inner tip portion defining an inner window, wherein a gap is defined between the inner tip portion and the outer tip portion,
   wherein the outer tube defines irrigation channels extending longitudinally from near the proximal end and configured to be arranged in fluid communication with the irrigation source, and further defines irrigation apertures configured to provide fluid communication between the irrigation channels and the gap at a position proximal to the outer window and the inner window.
14. The cutting assembly of claim 13, wherein the irrigation apertures are positioned proximal to the outer window.
15. The cutting assembly of claim 13 or 14, wherein the outer tube comprises a bend and the inner tube comprises a flexible region, wherein the irrigation apertures are positioned distal to the flexible region.
16. A cutting assembly configured to be coupled to an irrigation source, the cutting assembly comprising:
   a housing;
   an outer tube comprising a distal end, and a bend to angle the distal end relative to a longitudinal axis;
   an inner tube rotatably and coaxially disposed within the outer tube and comprising a flexible region traversing the bend of the outer tube; and
   a cutting tip secured to the inner tube and angled relative to the longitudinal axis,
   wherein the outer tube defines irrigation channels and configured to be in fluid communication with the irrigation source, wherein the irrigation channels are fluidly separated from the flexible region of the inner tube, and wherein the outer tube further defines irrigation apertures positioned distal to the flexible region and configured to provide fluid communication between the irrigation channels and a gap between the cutting tip and the outer tube.
17. The cutting assembly of claim 16, wherein the outer tube comprises an outer tip portion defining an outer window, and wherein the cutting tip defines an inner window such that the cutting assembly is a shaver.
18. The cutting assembly of claim 16, wherein the cutting tip is a bur head.
19. The cutting assembly of claim 13-18, wherein the outer tube comprises a bend, wherein the irrigation channels traverse the bend.
20. The cutting assembly of any one of claims 13-19, wherein the inner tube comprises segments defining the flexible region, wherein the irrigation channels are fluidly separated from the segments.
21. The cutting assembly of any one of claims 13-20, wherein the outer tube is monolithic, and the irrigation channels are encapsulated within the outer tube.
22. The cutting assembly of any one of claims 13-21, wherein further comprising a hypotube disposed within and coupled to the outer tube, wherein and the irrigation channels are defined between the outer tube and the hypotube.
23. The cutting assembly of any one of claims 13-22, wherein the irrigation apertures are recesses defined at a distal end of the irrigation channels.
24. The cutting assembly of any one of claims 13-23, wherein the irrigation channels are four irrigation channels radially spaced equally about the longitudinal axis, and wherein the irrigation apertures are four irrigation apertures radially spaced equally about the longitudinal axis.
25. The cutting assembly of any one of claims 13-24, wherein the housing defines an irrigation cavity, an aperture through which the inner tube extends, and recesses in fluid communication with the aperture and the irrigation channels.
26. The cutting assembly of claim 25, wherein the recesses of the housing are in a cruciform arrangement.

## Claims

1. A cutting assembly (10) configured to be coupled to an irrigation source and an aspiration source, the cutting assembly (10) comprising:
a housing (12);
an outer tube (16) comprising a proximal end coupled to the housing (12) and comprising an outer tip portion (36) defining an outer window (42); and
an inner tube (18) rotatably and coaxially disposed within the outer tube (16) and defining an aspiration lumen (24) configured to be in arranged in fluid communication with the aspiration source, the inner tube (18) comprising an inner tip portion (38) defining an inner window (46), wherein a gap (58) is defined between the inner tip portion (38) and the outer tip portion (36),
wherein the outer tube (16) defines irrigation channels (60) extending longitudinally from near the proximal end and configured to be arranged in fluid communication with the irrigation source, and further defines irrigation apertures (62) configured to provide fluid communication between the irrigation channels (60) and the gap (58) at a position proximal to the outer window (42) and the inner window (46).

2. The cutting assembly (10) of claim 1, wherein the irrigation apertures (62) are positioned proximal to the outer window (42).

3. The cutting assembly (10) of claim 1 or 2, wherein the outer tube (16) comprises a bend and the inner tube comprises a flexible region, wherein the irrigation apertures (62) are positioned distal to the flexible region.

4. A cutting assembly (10) configured to be coupled to an irrigation source, the cutting assembly (10) comprising:
a housing (12);
an outer tube (16) comprising a distal end, and a bend (52) to angle the distal end relative to a longitudinal axis;
an inner tube rotatably and coaxially disposed within the outer tube (16) and comprising a flexible region (54) traversing the bend of the outer tube (16); and
a cutting tip (26) secured to the inner tube (18) and angled relative to the longitudinal axis,
wherein the outer tube (16) defines irrigation channels (60) and configured to be in fluid communication with the irrigation source, wherein the irrigation channels (60) are fluidly separated from the flexible region (54) of the inner tube (18), and wherein the outer tube (16) further defines irrigation apertures (62) positioned distal to the flexible region (54) and configured to provide fluid communication between the irrigation channels (60) and a gap (58) between the cutting tip (26) and the outer tube (16).

5. The cutting assembly (10) of claim 4, wherein the outer tube (16) comprises an outer tip portion (36) defining an outer window (42), and wherein the cutting tip (26) defines an inner window (46) such that the cutting assembly (10) is a shaver.

6. The cutting assembly (10) of claim 4, wherein the cutting tip (26) is a bur head (84).

7. The cutting assembly (10) of claim 6, wherein the bur head (84) comprises a cutting element and defines an aspiration port (90) proximal to the cutting element.

8. The cutting assembly (10) of claim 6, further comprising an irrigation spacer (96) disposed within a cavity (98) defined by the housing (12), the irrigation spacer (96) comprising a hub defining a bore through which the inner tube (18) is rotatably disposed, and fins extending radially from the hub to be secured within the cavity (98).

9. The cutting assembly (10) of any one of claims 1-8, wherein the inner tube (18) comprises segments (56) defining the flexible region (54), wherein the irrigation channels (60) are fluidly separated from the segments (56).

10. The cutting assembly (10) of any one of claims 1-9, wherein the outer tube (16) is monolithic, and the irrigation channels (60) are encapsulated within the outer tube (16).

11. The cutting assembly (10) of any one of claims 1-10, further comprising a hypotube disposed within and coupled to the outer tube (16), wherein the irrigation channels (60) are defined between the outer tube (16) and the hypotube.

12. The cutting assembly (10) of any one of claims 1-11, wherein the irrigation apertures (62) are recesses defined at a distal end of the irrigation channels (60).

13. The cutting assembly (10) of any one of claims 1-12, wherein the irrigation channels (60) are four irrigation channels (60) radially spaced equally about the longitudinal axis, and wherein the irrigation apertures (62) are four irrigation apertures (62) radially spaced equally about the longitudinal axis.

14. The cutting assembly (10) of any one of claims 1-13, wherein the housing (12) defines an irrigation cavity (30), an aperture (66) through which the inner tube (18) extends, and further defines recesses (68) in fluid communication with the aperture (66) and the irrigation channels (60).

15. The cutting assembly (10) of claim 14, wherein the recesses (68) of the housing (12) are in a cruciform arrangement.
